# EUROPEAN PATENT SPECIFICATION

(11) **EP 2 749 556 B1**
(45) Date of publication and mention of the grant of the patent: **25.07.2018**
(21) Application number: 14000056.3
(22) Date of filing: 02.01.2014
(51) Int. Cl.: C07D 279/28, A61K 31/5415, A61P 25/18

(54) **An improved process for the preparation of pure cyamemazine and salts thereof**
Verbessertes Verfahren zur Herstellung von reinem Cyamemazin und Salze davon
Procédé amélioré pour la préparation de cyamémazine pure et ses sels

(30) Priority: 24.12.2012 IN CH54342012
(43) Date of publication of application: 02.07.2014
(73) Proprietor: Micro Labs Limited, Bangalore 560 001 (IN)
(72) Inventor: Annem, Chandrahasa Reddy, 560 105 Bangalore (IN); Patel, Pareshkumar Kashavial, 560 106 Bangalore (IN); Kumar, Pramod, 560 105 Bangalore (IN); Gajula, Madhusudana Rao, 560 105 Bangalore (IN); Alla, Srinivasa Reddy, 560 105 Bangalore (IN)
(74) Representative: Høiberg P/S

(56) References cited:
- US-A- 2 877 224
- G SINGH ET. AL.: "N-Demythylation of Cyamemazine via non-classical Polonovski reaction and its conjugation to bovine serum albumin.", BIOORGANIC AND MEDICINAL CHEMISTRY LETTERS, vol. 22, 4 February 2012 (2012-02-04), pages 2160-2162, XP002724408,
- A. HAMEG ET. AL.: "Affinity of cyamemazine, an anxiolytic antipsychotic drug, for human recombinant dopamine vs. serotonin receptor subtypes.", BIOCHEMICAL PHARMACOLOGY, vol. 65, no. 3, 1 August 2003 (2003-08-01) , pages 435-440, XP002724409,
- P. N. CRAIG ET. AL.: "Synthesis of Phenothiazines. VI. Certain 2-Substituted Phenothiazines and Their 10-Aminoalkyl Derivatives.", JOURNAL OF ORGANIC CHEMISTRY, vol. 26, no. 4, 1 April 1961 (1961-04-01), pages 1138-1143, XP002724410,
- "CYAMEMAZINE", SANTA CRUZ BIOTECH , XP002724411, Retrieved from the Internet: URL:www.scbt.com/datasheet-211144.html [retrieved on 2014-05-15]

## Description

### FIELD OF THE INVENTION:

The present invention is in the field of chemistry and more particularly the invention deals with an improved process for the preparation of cyamemazine of formula-1 or its pharmaceutically acceptable salt thereof.

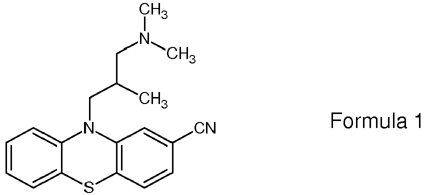

### BACKGROUND OF THE INVENTION:

Cyamemazine is chemically known as 10-(3-dimethylamino-2-methyl-propyl) phenothiazine-2-carbonitrile, in the form of a base or of a pharmaceutically acceptable salt, and in particularly tartrate salt thereof, is a neuroleptic of the phenothiazine type (U.S. Pat. No. 2,877,224). The phenothiazine derivative, cyamemazine is the most widely used antipsychotic neuroleptic drug in France. It is available under the brand name of Tercian®. It is used in the symptomatic treatment of anxiety in all its forms and optionally can be combined with an antidepressant in conditions of serious depression. This medicine is used in order to treat schizophrenia and lot of psychoses.

Various methods for the preparation of cyamemazine are known in the art, first disclosed in U.S. Pat. No. 2,877,224; Cyanophenthiazine was treated with 1-dimethylamino-2-methyl-3-chloropropane in the presence of condensing agent such as sodamide in an aromatic hydrocarbon solvent such as xylene to obtain cyamemazine as a residue, further converting it into the form of acid addition salts such as maleate and re-crystallised with ethanol (Example 2). This process which involves several disadvantages such as unfair condensation reaction takes longer time to complete (about 20 to 24 hours), superfluous color, due to more impurities formation and there by generating a semisolid material. Hence it requires repeated purification, which effects on overall yield of the final product of cyamemazine salts (∼38%). Further, this patent does not describe any crystallographic information.

Other methods do not envisage recovery of the intermediates; as a consequence, the quality of the cyamemazine maleate obtained is poor and requires further purification. Cyamemazine tartrate salt is not being formed from the crude cyamemazine due to high content of unexpected impurities. Moreover, distinctively cyamemazine tartrate salts and process is not available in the literature.

However, there is a unmet need to develop an improved process for the preparation of the cyamemazine or acid addition salts of the formula-1, which is commercially viable and which has advantage over the processes as described in the prior art documents.

### OBJECTS OF THE INVENTION:

An object of the present invention is to provide a simple and efficient process for the preparation of pure cyamemazine of formula-1 or its pharmaceutically acceptable salts thereof, which is commercially viable and more economical.

Another objective of the present invention is to provide processes to prepare stable, reproducible crystalline and pure forms of cyamemazine, cyamemazine maleate and cyamemazine tartrate with good yield and acceptable color.

Another objective of the present invention is to prepare substantially pure crystalline cyamemazine of formula-1 or its pharmaceutically acceptable salts thereof, which employs less time consuming with unproblematic, by avoiding impurities (especially dimer impurities such as X and Y) in the reaction.

### SUMMARY OF THE INVENTION:

In one embodiment the present inventors have proceeded with extensive research. As a result, it is to provide an improved process for the preparation of pure cyamemazine of formula-1 or its pharmaceutically acceptable salts thereof, through various salts of cyamemazine.

In another embodiment, the present invention is to provide an improved process for the preparation of cyamemazine or salts thereof, which comprising of: a) reacting 2-cyanophenthiazine of the formula-2 or salt thereof, with 1-dimethylamino-2-methyl-3-chloropropane of formula-3 or salt thereof, in the presence of base and phase transfer catalyst in organic solvent or mixture thereof, to produce the cyamemazine of formula-1; which is commercially viable and more economical.

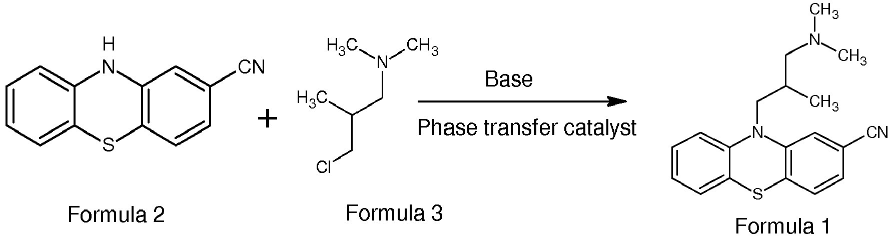

In another embodiment, the invention encompasses to prepare substantially pure crystalline cyamemazine or cyamemazine maleate salt or cyamemazine tartrate salt by avoiding impurities in the reaction like dimer impurities X and Y.

In another embodiment, the invention encompasses a novel pure crystalline form of cyamemazine, referred herein as form C.

In another embodiment, the invention encompasses a pure crystalline form of cyamemazine maleate, referred herein as form M.

In another embodiment, the invention encompasses a novel pure crystalline form of cyamemazine tartrate, referred herein as form T.

In another embodiment, the invention encompasses a process for preparing cyamemazine maleate or cyamemazine tartrate by the process of the present invention and converting it to pure cyamemazine.

In another embodiment, the invention to prepare substantially pure crystalline cyamemazine of formula-1 or its pharmaceutically acceptable salts thereof, in accordance with and using the conventional methods of chemical synthesis; which is schematically presented below:

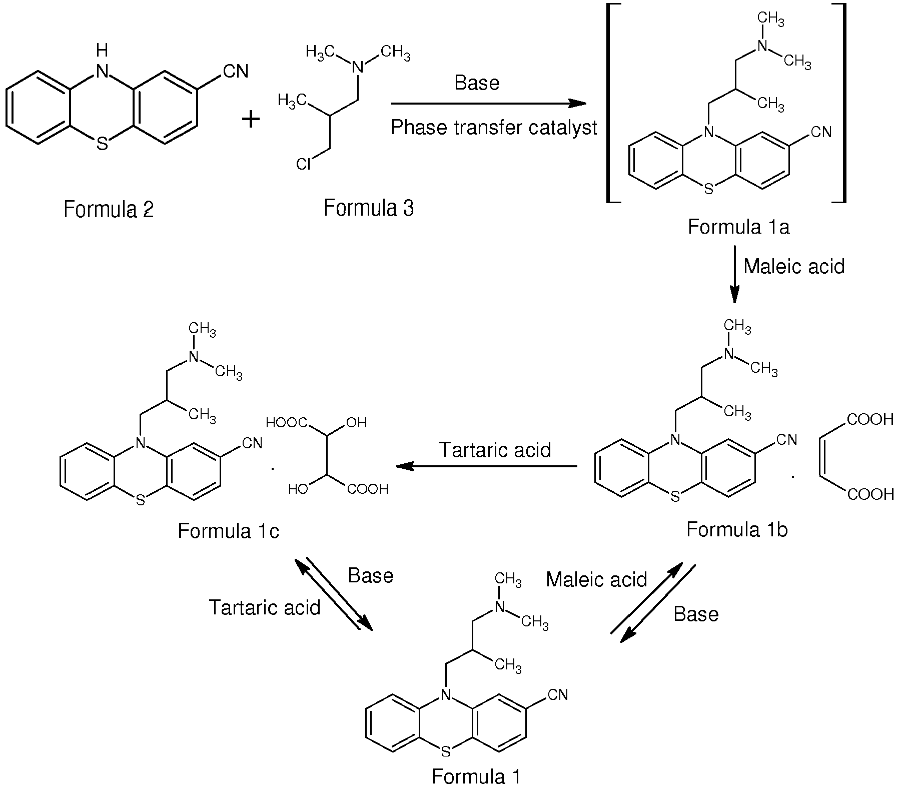

Further, it is the aim of the invention to provide a process for the preparation of cymemazine of formula-1 and its pharmaceutically acceptable salts, prepared according to instant invention.

### BRIEF DESCRIPTION OF THE FIGURES

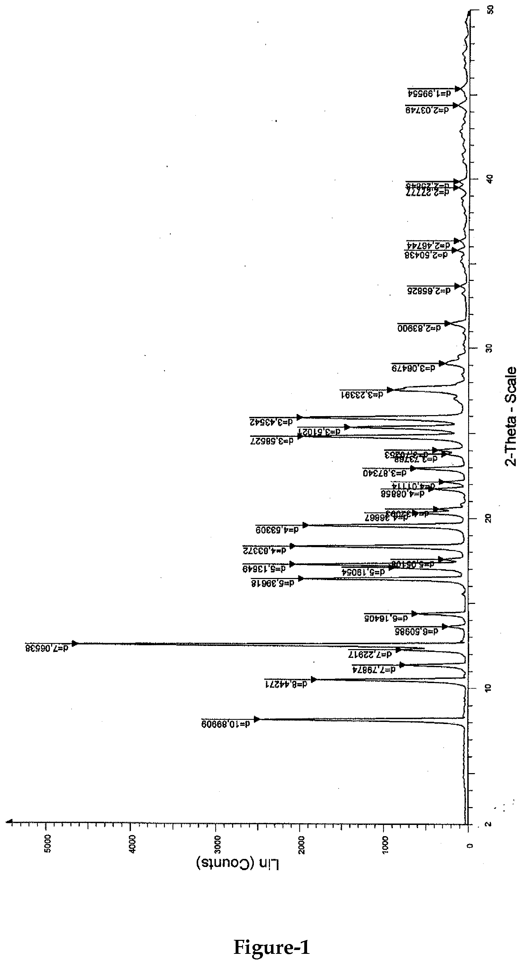

FIG. 1 describes the XRPD of the crystalline form C of Cyamemazine prepared by the process of the present invention.

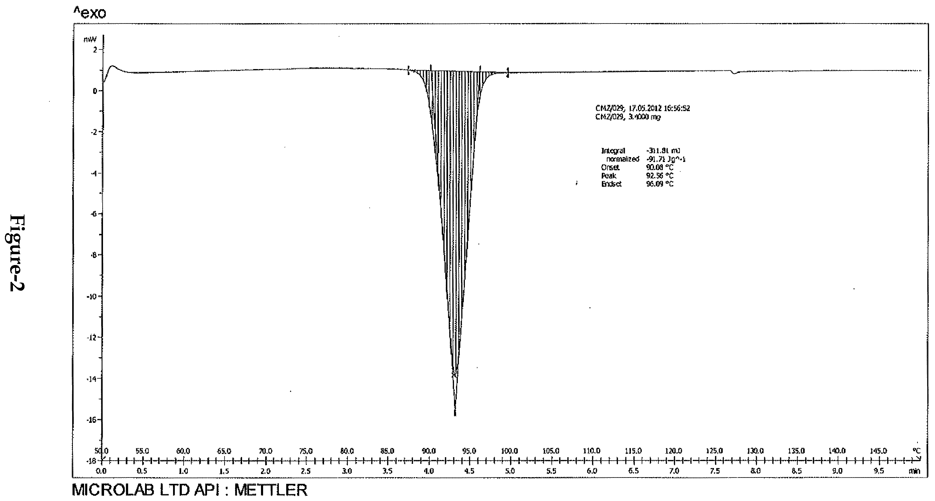

FIG. 2 describes the DSC of the crystalline form C of Cyamemazine prepared by the process of the present invention.

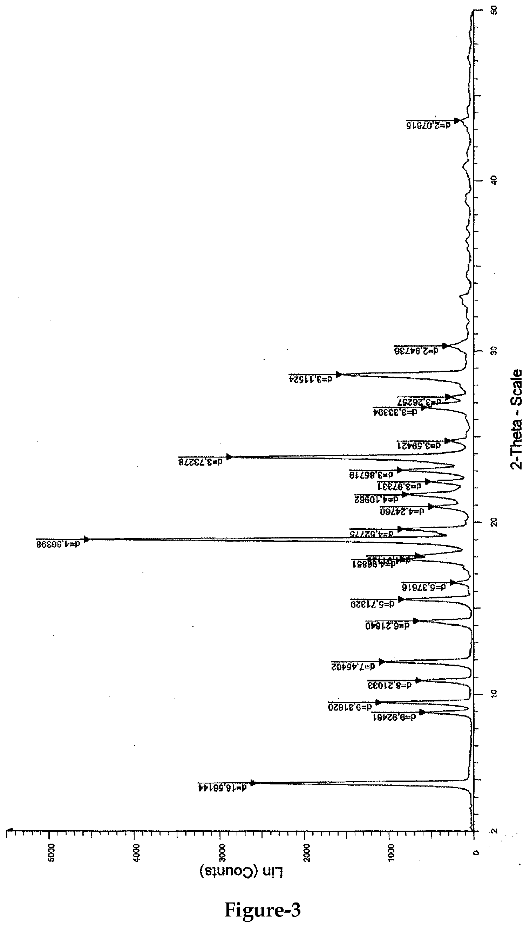

FIG. 3 describes the XRPD of the crystalline form M of Cyamemazine maleate prepared by the process of the present invention.

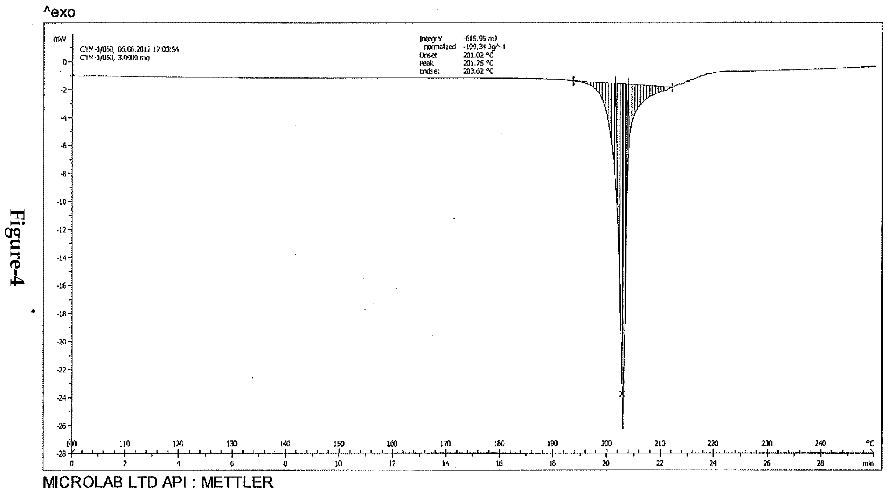

FIG. 4 describes the DSC of the crystalline form M of Cyamemazine maleate prepared by the process of the present invention.

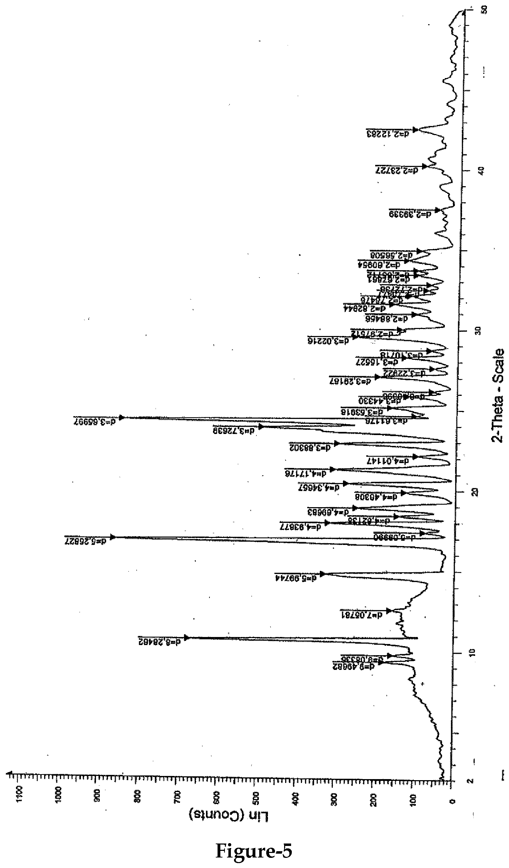

FIG. 5 describes the XRPD of the crystalline form Tof Cyamemazine tartrate prepared by the process of the present invention.

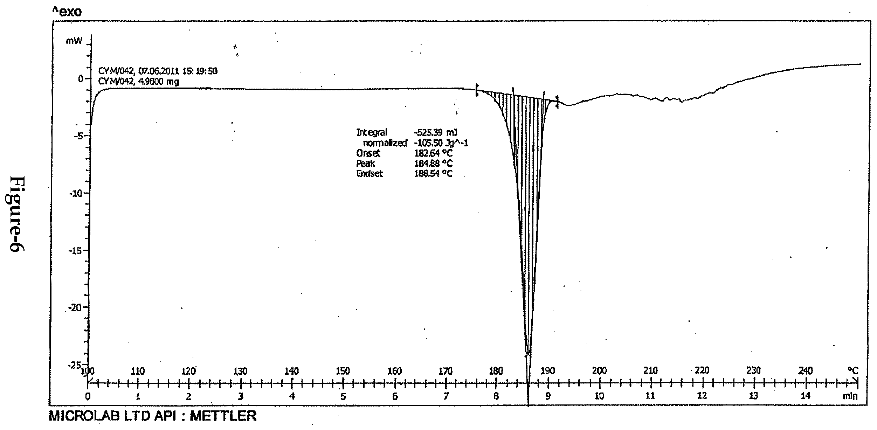

FIG. 6 describes the DSC of the crystalline form T of Cyamemazine tartrate prepard by trhe process of the present invention.

### DETAILED DESCRIPTION OF THE INVENTION:

Accordingly, the present invention provides an improved process for the preparation of highly pure cyamemazine of formula-1 or pharmaceutically acceptable salt thereof, with good yield, through various salts of cyamemazine.

According to the present invention, the process for the preparation of cyamemazine of formula-1 or pharmaceutically acceptable salt thereof, comprising: 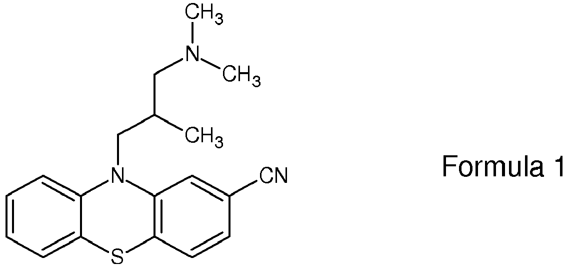
a) reacting 2-cyanophenthiazine of the formula-2 or salt thereof, with 1-dimethylamino-2-methyl-3-chloropropane of formula-3 or salt thereof, in the presence of base and phase transfer catalyst in organic solvent or mixture thereof, to produce the cyamemazine of formula-la 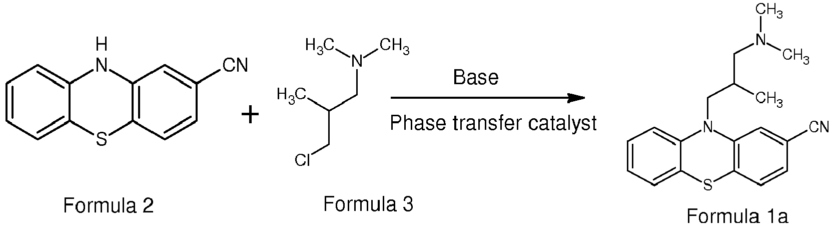
b) reacting the compound of formula-la with an acid in organic solvent or mixture thereof, to obtain cyamemazine acid salts
c) optionally purifying the step (b) and reacting with base to get the pure cyamemazine
d) optionally isolating the cyamemazine and optionally reacting with suitable salts to converting the cyamemazine of formula-1 or pharmaceutically acceptable salt thereof.

According to the present invention, the process for the preparation of cyamemazine tartrate of formula-1c, comprising 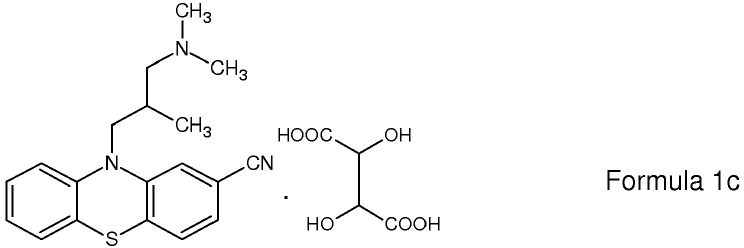
a) reacting 2-cyanophenthiazine of the formula-2 or salt thereof, with 1-dimethylamino-2-methyl-3-chloropropane of formula-3 or salt thereof, in the presence of base and phase transfer catalyst in organic solvent or mixture thereof, to produce the cyamemazine of formula-la 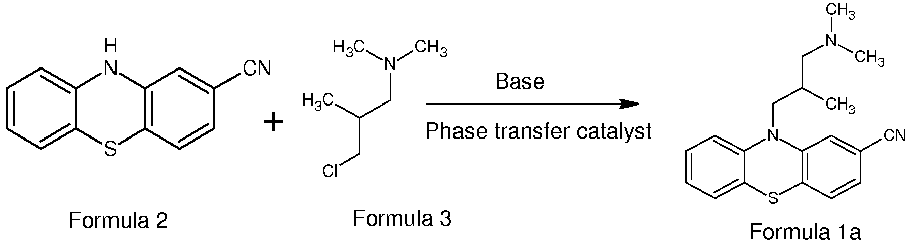
b) reacting the compound of formula-la with maleic acid in organic solvent or mixture thereof, to obtain a cyamemazine maleate salt
c) optionally reacting with base to get cyamemazine
d) optionally isolating or purifying the cyamemazine maleate salt or cyamemazine and further reacting with tartaric acid to obtain the cyamemazine tartrate salt.

According to the present invention, the process for the preparation of cyamemazine of formula-1, comprising: 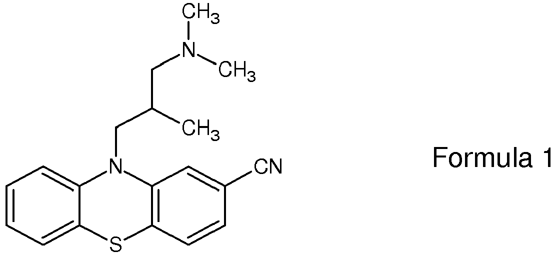 reacting cyamemazine acid salts of formula-1 with base to get the pure cyamemazine of formula-1.

According to the present invention, the process for the preparation of cyamemazine of formula-1 or salts thereof, comprising: 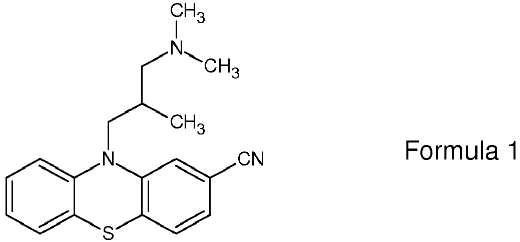
a) reacting 2-cyanophenthiazine of the formula-2 or salt thereof, with 1-dimethylamino-2-methyl-3-chloropropane of formula-3 or salt thereof, in the presence of base and phase transfer catalyst in organic solvent or mixture thereof, to produce the cyamemazine of formula-1 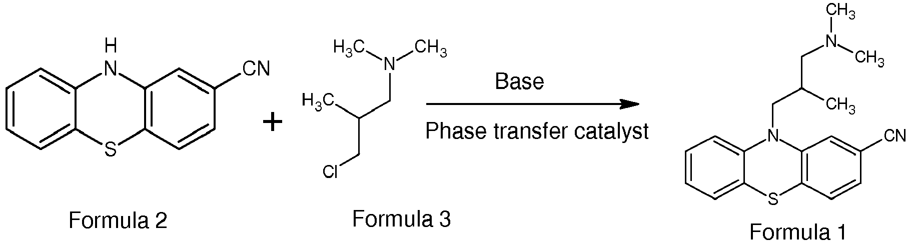
b) optionally purifying or converting the salts of the step (a) to get the pure cyamemazine of formula I.

According to the present invention, the phase transfer catalyst used in the condensation reaction, selected from the group such as alkylammonium halides like tetrabutyl ammonium bromide, tetrapropyl ammonium bromide, benzyltributyl ammonium chloride, tetraethyl ammonium bromide, tetraoctylammonium bromide, benzyltrimethylammonium chloride, benzyltriethylammonium chloride, tetrabutylammonium iodide, tetrabutyl ammonium tribromide, tetrabutylammonium bromide, tetraheptylammonium bromide; alkyl phosphonium halides like ethyltriphenylphosphonium bromide, ethyltriphenyl phosphonium iodide, carboxybutyltriphenylphosphonium bromide, benzyltriphenyl phosphonium bromide, benzyltriphenylphosphonium chloride; tetrapropylammonium hydroxide; tetrabutylammonium hydrogen sulfate; tetrabutylammonium hexafluoro phosphate; tetrabutylammonium acetate; and cetylpyridinium chloride, etc; preferably the phase transfer catalyst is tetrabutylammonium bromide.

According to the present invention, the base used in the condensation reaction, selected from the group such as organic or inorganic bases; organic bases such as amines like triethylamine, diethylamine, monomethylamine, and benzylamine, etc; preferably inorganic bases such as alkali metal hydroxides, sodium, potassium or ammonium hydroxides and alkali metal carbonates such as sodium, potassium or ammonium carbonates, alkali metal bi-carbonates such as sodium, potassium or ammonium bicarbonates etc, most preferably sodium hydroxide.

According to the present invention, the base used in the condensation reaction is about an equimolar amount to about 2 times with respect to compound of formula-2. Preferably about 1.5 to 2 equivalents are used.

In condensation reaction the organic solvent is preferably selected from aromatic hydrocarbons such as toluene, xylene, etc; more preferably, the organic solvent selected is toluene.

The condensation reaction is well carried out for a period of about 2 to 3 hours with phase transfer catalysts. Preferably about 2 hours 30 minutes. In the absence of phase transfer catalyst, the reaction will take about 10 to 15 hours to complete as per present invention.

As per the prior art process disclosed in U.S. Pat. No. 2,877,224; 2-Cyanophenthiazine of the formula-2 is condensed with 1-dimethylamino-2-methyl-3-chloropropane of formula-3 in the presence of xylene solvent and sodamide at 140° C, which is not suitable condition for commercial scale and the reaction takes longer time and the formation of unwanted impurities are more, finally affords low yield and purity.

In the present invention, the condensation reaction can be carried out particularly at temperature 100 to 110° C then the reaction is smoothly proceeded with high yield and low impurities. Preferably, the reaction is carried out at a temperature about 105° C. At less than 100° C, the reaction does not proceed, if the temperature is higher than 110° C, then impurities formation is very high such as impurity X and Y.

In the said condensation reaction, the product of cyamemazine compound of formula-la is not isolated as a solid and hence it's directly converted to a suitable acid addition salts; preferably maleic acid. Especially the direct conversion of tartrate salts affords more impurities formation and low yield. Thus first conversation to maleate salt followed by saltification to tartrate affords higher yield and purity.

The present invention disclosed the term as "salts" are pharmaceutically acceptable acid salts includes, but is not limited to mineral acids or inorganic acids or organic acids such as acetic acid, adipic acid, ascorbic acid, benzenesulfonic acid, benzoic acid, cinnamic acid, citric acid, cyclamic acid, dodecylsulfuric acid, ethanesulfonic acid, formic acid, fumaric acid, galactaric acid, gentisic acid, glutamic acid, glutaric acid, glycerophosphoric acid, glycolic acid, hippuric acid, hydrobromic acid, hydrochloric acid, isobutyric acid, lactic acid, lactobionic acid, lauric acid, maleic acid, malonic acid, methanesulfonic acid, nitric acid, oleic acid, oxalic acid, palmitic acid, pamoic acid, phosphoric acid, propionic acid, salicylic acid, sebacic acid, stearic acid, succinic acid, sulfuric acid, tartaric acid, thiocyanic acid, toluenesulfonic acid, undecylenic acid, etc; more preferably, the salts are maleic acid and tartaric acid salts.

The cyamemazine acid addition salt compound of formula-1 is converted to cyamemazine, using the suitable bases includes, but is not limited to organic or inorganic bases; organic bases such as amines like triethyl amine, diethyl amine, benzylamine, pyridine, aniline etc; inorganic bases such as alkali metal hydroxides, sodium, potassium or ammonium hydroxides and alkali metal carbonates such as sodium, potassium or ammonium carbonates alkali metal bi-carbonates such as sodium, potassium or ammonium bicarbonates etc, more preferably sodium hydroxide.

The compound of formula-la is converted to salts of formula-1 and further converted to pure cyamemazine by using the organic solvent preferably selected, but is not limited to alcoholic solvents such as branched or chained C₁-C₄ selected alcohols such as methanol, ethanol, isopropyl alcohol, etc; ethers such as diethyl ether, diisopropyl ether, methyl tertiary-butyl ether, tetrahydrofuran, dioxane etc; aliphatic hydrocarbons such as C₁-C₁₀ straight chain or branched hydrocarbons such as *n*-hexane, *n*-heptane, cyclohexane, pentane, etc; and aromatic hydrocarbons such as toluene, xylene, etc; haloalkanes such as dichloromethane, chloroform, etc; ketones, water and mixtures thereof. Preferably, the organic solvent is selected from ketones, ethers, and esters; more preferably the solvent is acetone or diisopropyl ether or ethyl acetate.

According to the present invention, which relates to processes for preparing pure solid compounds of cyamemazine, cyamemazine maleate, cyamemazine tartrate have the purity greater than 98%, preferably greater than 99%, more preferably greater than 99.9%.

The present invention provides an additional new aspect in the development to reduce the dimer impurities compound of formula-X and formula-Y, these dimer impurities are not known in the art and it's very difficult to minimize in the process. To overcome this negative aspect, these impurities are isolated, characterized and controlled in the present invention of "An improved process for the preparation of pure cyamemazine and salts thereof". The present invention contains less than about 0.02% area by HPLC of one or more of the impurities X, and Y. The formed dimer impurities were removed by treating the reaction mass with acetone or methanol solvents and mixture thereof.

Impurity X is 10-10'-biphenothiazine-2-carboxamide.

Impurity Y is 7-(2-cyano-10*H*-phenothiazin-10-yl)-10-(3-(dimethylamino)-2-methyl propyl)-10*H*-phenothiazine-2-carbonitrile.

Impurity X and Y are respectively represented by formula-X and formula-Y. Accordingly, the present invention provides a novel compound of formula-X

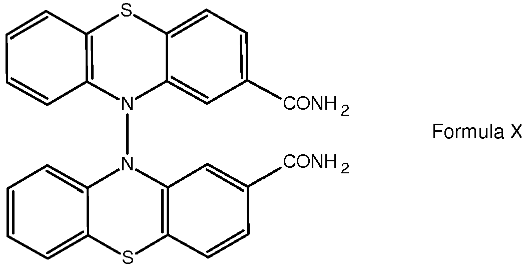

Accordingly, the present invention provides a novel compound of formula-Y

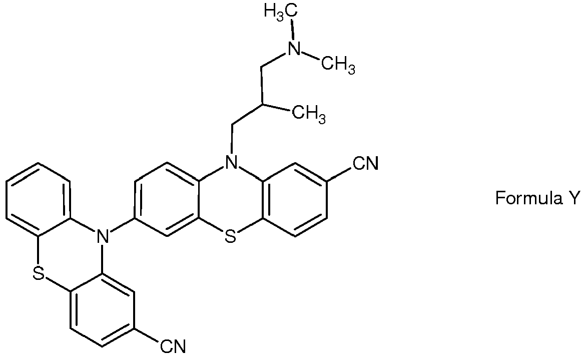

The impurities X and Y are formed during the condensation of 2-cyanophenthiazine of the formula-2 with 1-dimethylamino-2-methyl-3-chloro propane of formula-3 reaction described previously.

Isolation of the impurity X is described as follows:
After completion of condensation reaction, water was added to the reaction mass, separated solid was filtered. The material was re-crystallized in acetone and then further purified by column chromatography (Purity >99.5% by HPLC; M.P. 225-245° C). ¹H-NMR (DMSO, 400 MHz): δ 7.30-6.65 (m, 14H), 7.81 (s, N*H*₂), 8.68 (s, N*H*₂); ¹³C-NMR (DMSO, 100.6 MHz): δ113.55, 114.53, 115.66, 120.27, 120.47, 121.94, 125.69, 126.21, 127.74, 133.80, 141.56, 141.85, 167.43; MS *m*/*z* : 242 ([M/2]+1); IR (neat, cm⁻¹): 3370, 3320, 3143, 1707, 1599, 1562, 1472, 1437, 1417, 1114.

Isolation of the impurity-Y is described as follows:
After completion of condensation reaction, water was added to the reaction mass, separated solid was filtered. The filtrate was distilled, the residue was dissolved in acetone and added methanolic maleic acid solution. The reaction mass was stirred for 1 hour at reflux temperature, cooled to 20 to 30° C and maintained for 30 minutes. The filtrate was distilled and the impurity-Y was isolated in hexane, further separated by column chromatography (Purity >99.5% by HPLC; M.P. 261-265° C). ¹H-NMR (DMSO, 300 MHz): δ 0.95 (s, 3H, C*H₃*)*,* 2.12 (s, 7H, C*H₃,* C*H₃,* C*H*), 2.20-2.40 (m, 1H), 2.80-3.00 (m, 1H), 3.98-4.01 (m, 2H), 6.12 (d, 1H, *J* = 6), 6.29 (s, 1H), 6.80-7.60 (m, 11H); ¹³C-NMR (DMSO, 75.46 MHz): δ16.66, 28.68, 45.62, 45.75, 51.52, 63.49, 109.51, 110.34, 116.14, 117.13, 117.52, 118.50, 118.70, 119.00, 119.35, 123.39, 126.05, 126.16, 126.43, 126.49, 126.64, 127.38, 127.91, 128.14, 129.21, 130.16, 131.00, 134.06, 142.43, 143.93, 144.52, 145.39; MS *m*/*z* (%): 546.6 (M+1); IR (neat, cm⁻¹): 3433, 3379, 3061, 2225, 1463, 1400, 1103, 1031.

The present invention provides a process for preparing cyamemazine or its salts thereof; containing less than 0.15 % area by HPLC of one or more of the impurities X and Y. Preferably, the amount of each of the two impurities in the sample of final is less than about 0.02%.

The amounts of X and Y are also measured by HPLC. An exemplary impurity profile determination is describes as, the related substances of residue compound of formula-la (cyamemazine or its salts thereof) measured using HPLC with the following chromatographic conditions: column: Phenomenex Gemini (C-18, 250mm x 4.6mm, 5µm) or equivalent column using mobile phase (Mobile phase-A: add 1 mL glacial acetic acid in 1000 mL of water, add 8 g of 1-octane sulphonic acid sodium salt, sonicate to dissolve; Mobile phase-B: acetonitrile; Mobile phase-C: methanol) with flow rate 1.0 mL/min. The detection limit in the HPLC method is 0.01%. RRT of impurity-X∼ 0.58 and impurity-Y∼ 2.40.

The present invention provides processes to prepare solid state forms of substantially pure crystalline cyamemazine, referred to herein as form C characterized by an X-ray diffraction pattern having peaks at 2- theta values 8.1, 10.4, 12.5, 16.4, 17.2, 18.3, 19.5, 24.8, 25.3, and 25.9 ± 0.2° 2-theta.

The present invention provides a process to prepare solid state forms of substantially pure crystalline cyamemazine, referred to herein as form C characterized by an X-ray diffraction pattern having peaks at 2-theta values 8.1, 11.3, 10.4, 12.2, 12.5, 14.3, 16.4, 17.2, 17.0, 18.3, 19.5, 20.3, 22.9, 24.8, 25.3, 25.9 and 27.9 ± 0.2° 2-theta.

The present invention provides a process to prepare crystalline form C of cyamemazine characterized by an X-ray diffraction pattern substantially as shown in Fig-1.

Preferably the cyamemazine crystalline form C of present invention is further characterized by a DSC with an endothermic peak at 92.5° C, as shown in Fig-2, as measured by a Differential Scanning Calorimeter (DSC) at a scan rate of 10° C per minute.

The present invention provides a process to prepare solid state forms of substantially pure crystalline cyamemazine maleate, referred to herein as form M characterized by an X-ray diffraction pattern having peaks at 2-theta values 4.7, 19.0, 23.8 and 28.6 ± 0.2° 2-theta. The crystalline is similarly produces as per process described in U.S. Pat. No. 2,877,224 described in Example 2.

The present invention provides a process to prepare solid state forms of substantially pure crystalline cyamemazine maleate, referred to herein as form M characterized by an X-ray diffraction pattern having peaks at 2-theta values 4.7, 8.9, 9.4, 10.7, 11.8, 14.2, 15.4, 17.8, 18.0, 19.0, 19.5, 20.8, 21.6, 22.3, 23.0, 23.8, 26.7 and 28.6 ± 0.2° 2-theta.

The present invention provides a process to prepare crystalline form M of cyamemazine maleate characterized by an X-ray diffraction pattern substantially as shown in Fig-3.

Preferably the cyamemazine maleate crystalline form M of present invention is further characterized by a DSC with an endothermic peak at 201.7° C, as shown in Fig-4, as measured by a Differential Scanning Calorimeter (DSC) at a scan rate of 5° C per minute.

The present invention provides a process to prepare solid state forms of substantially pure crystalline cyamemazine tartrate, referred to herein as form T characterized by an X-ray diffraction pattern having peaks at 2- theta values 10.6, 14.7, 16.8, 17.9, 20.4, 21.2, 22.8, 23.8 and 24.2 ± 0.2° 2-theta.

The present invention provides a process to prepare solid state forms of substantially pure crystalline cyamemazine tartrate, referred to herein as form T characterized by an X-ray diffraction pattern having peaks at 2-theta values 9.3, 9.7, 10.6, 12.5, 14.7, 16.8, 17.9, 18.3, 18.8, 20.4, 21.2, 22.8, 23.8, 24.2, 25.1, 27.0, 28.2, 29.5, 30.0, 31.5 and 34.3 ± 0.2° 2-theta.

The present invention provides a process to prepare crystalline form T of cyamemazine tartrate characterized by an X-ray diffraction pattern substantially as shown in Fig-5.

Preferably the cyamemazine tartrate crystalline form T of present invention is further characterized by a DSC with an endothermic peak at 184.8° C, as shown in Fig-6, as measured by a Differential Scanning Calorimeter (DSC) at a scan rate of 10° C per minute.

According to the present invention, encompasses the use of any one, or a combination of the above described crystalline forms of cyamemazine maleate and cyamemazine tartrate for the preparation of cymemazine.

According to the present invention, cyamemazine encompasses with high purity which is manufactured from cyamemazine tartrate, which is manufactured from cyamemazine maleate. Comparatively cyamemazine is manufactured independently form cyamemazine maleate.

The term compound of cyamemazine of formula-1 used herein is inclusive of all polymorphic forms of cyamemazine or its pharmaceutically acceptable salt thereof, for example polymorphs of crystalline form, or hydrates, or solvates thereof.

Differential Scanning Calorimeter (DSC) was performed on Mettler-Toledo; DSC-1/500/1070 instrument. Samples of 2 mg to 3 mg weighed in aluminum crucible with holes were scanned at a heating rate of 5° C or 10° C per minute under Nitrogen atmosphere at a rate of 10 mL/min.

The present invention can be used, further to provide a pharmaceutical composition containing cyamemazine and its pharmaceutically acceptable salts.

The invention is illustrated by the following examples which are only meant to illustrate the invention and not act as limitations.

### Reference Examples

### Preparation of Cyamemazine maleate according to US2877224 example 2:

A solution of 2-cyanophenothiazine (10 g) in anhydrous xylene (75 mL) was heated at reflux and treated with 95 % sodamide (2.15 g). The reaction was maintained for one hour and then a solution of 1-dimethylamino-2-methyl-3-chloropropane (7.05 g) in xylene (70 mL) was added over 15 minutes. The mixture was heated under reflux for 20 hours and then cooled. The reaction mixture was treated with water (40 mL) and methane sulphonic acid (75 mL). The xylene phase was removed and the aqueous phase was made alkaline with sodium hydroxide. The free base obtained was extracted with ether and ethereal extracts were dried over anhydrous potassium carbonate and concentrated to dryness. The residue was distilled in vacuum. 3-cyano-10-(3-dimethylamino-2-methylpropyl) phenothiazine (8.5 g) (B.P. 180-205° C / 0.9 mm Hg) is obtained. The acid maleate prepared in and recrystallised from ethanol melts at 204-205° C.

### Example-1

### Preparation of cyamemazine maleate

2-Cyanophenthiazine (100 g) was suspended in (700 mL) toluene and then added sodium hydroxide (30 g), TBAB (1 g) under stirring subsequently added the 1-dimethylamino-2-methyl-3-chloropropane (68 g). Then the reaction mass was raised at 100° C and stirred for 2 hours. The reaction mass was cooled to room temperature, quenched with water (400 mL) and filtered. The organic layer was collected and washed with water then distilled out the solvent under vacuum. Obtained the cyamemazine base of a dark-brown oil; B.P. 200-210° C.

The above oil was dissolved in acetone (980 mL) and added methanolic maleic acid solution (420 mL: 52 g). The reaction mass was stirred for 2 hours at reflux temperature and cooled to 20 to 30°C and maintained for 2 hours. The solid was filtered and washed with acetone. The above crude cyamemazine maleate was suspended in mixture of acetone (980 mL) and methanol (420 mL) was heated to reflux for 2 hours. The reaction mass was cooled to 25 to 30° C and maintained for 2 hours then filtered and washed the cake with acetone (200 mL) and dried at 50 to 60° C for 4 hours to obtain yellow crystalline powder of pure cyamemazine maleate (125 gm); M.P. 200-205° C.

### Example-2

### Preparation of cyamemazine tartrate

Cyamemazine maleate (100 g) was suspended in (700 mL) ethyl acetate and (500 mL) water and then the reaction mixture was stirred for 10 minutes. The pH of the reaction mass was adjusted to 7.5 - 8.5 with 10% sodium bicarbonate solution and stirred for 1 hour at room temperature. The organic layer was collected and washed with water then distilled out under vacuum. Obtained residue was dissolved in methanol (500 mL) and added methanolic tartraic acid solution (500 mL: 35 g). The reaction mass was stirred for 1 hour at reflux temperature and cooled to 20 to 30° C and maintained for 30 minutes. The solid was filtered and dried at 60 to 65° C for 4 hours to obtain yellow crystalline powder of cyamemazine tartrate (90 gm); M.P. 180-190° C.

### Example-3

### Preparation of cyamemazine

Cyamemazine tartrate (100 g) was suspended in (800 mL) di-isopropyl ether and (800 mL) water and then the reaction mixture was stirred for 10 minutes. The pH of the reaction mass was adjusted to 8-9 with ammonium solution and stirred for 1 hour at room temperature. The organic layer was separated and washed with water. Distilled out the solvent up to 30% under vacuum and cooled the reaction mass to 20 to 30° C and maintained for 1 hour. The solid was filtered and dried at 50 to 60° C under vacuum for 4 hours to obtain yellow crystalline powder of cyamemazine (60 gm); M.P. 90-95° C.

### Example-4

### Preparation of cyamemazine.

Cyamemazine tartrate (100 g) was suspended in (600 mL) ethyl acetate and (500 mL) water and then the reaction mixture was stirred for 10 minutes. The pH of the reaction mass was adjusted to 7-8 with 10% sodium bicarbonate solution and stirred for 1 hour at room temperature. The organic layer was collected and washed with water and then distilled out under vacuum. Obtained residue was dissolved in acetone (300 mL) and added water (1000 mL) drops by drop in not less than 2 hours. The reaction mass was stirred for 1 hour at 55 to 60° C and cooled to 20 to 30° C and maintained for 1 hour. The solid was filtered and dried at 50 to 60° C for 4 hours to obtain yellow crystalline powder of cyamemazine (64 gm); M.P. 90-95° C.

### Example-5

### Preparation of cyamemazine maleate

2-Cyanophenthiazine (100 g) was suspended in (800 mL) toluene and then added sodium hydroxide (30 g), TBAB (1 g) under stirring followed by 1-dimethylamino-2-methyl-3-chloropropane hydrochloride (90 g) and water (10 mL). Then the reaction mass temperature was raised at 100° C and stirred for 2 hours. After completion of the reaction, the reaction mass was quenched with water (700 mL) and filtered. The organic layer was collected and washed with water and then distilled out under vacuum. Obtained the dark-brown oily residue. The residue was dissolved in acetone (900 mL) and added methanolic maleic acid solution (400 mL: 52 g). The reaction mass was stirred for 1 hour at reflux temperature and cooled to 20 to 30° C and maintained for 30 minutes. The solid was filtered and dried at 50 to 60° C for 4 hours to obtain yellow crystalline powder of cyamemazine maleate (135 gm); M.P. 200-205° C.

### Example-6

### Preparation of cyamemazine

In a solution of sodium hydroxide (6 g) in toluene (140 mL), added 2-cyanophenthiazine (20 g) followed by TBAB (0.2 g) and toluene (20 mL) solution of 1-dimethylamino-2-methyl-3-chloropropane (13.6 g) under stirring. Then the reaction mass was maintained for 2 hours at 100° C. The reaction mass was cooled to room temperature, quenched with water (80 mL) and the solid was filtered off. The filtrate was washed with water and then concentrated under vacuum. Obtained residue was cooled to 25-30° C and stirred with toluene (10 mL) for 12 hours at 0-5 ° C to obtain the cyamemazine.

### Example-7

### Preparation of cyamemazine

Cyamemazine maleate (100 g) was suspended in (800 mL) di-isopropyl ether and (800 mL) water and then the reaction mixture was stirred for 10 minutes. The pH of the reaction mass was adjusted to 8-9 with ammonium solution and stirred for 1 hour at room temperature. The organic layer was separated and washed with water. Distilled out the solvent up to 30% under vacuum and cooled the reaction mass to 20 to 30° C and maintained for 1 hour. The solid was filtered and dried at 50 to 60° C under vacuum for 4 hours to obtain yellow crystalline powder of cyamemazine (65 gm); M.P. 90-95° C.

The invention can in one embodiment be characterized by the following set of items. These items should not be considered as claims.
1. A process for the preparation of cyamemazine of formula-1 or pharmaceutically acceptable salts thereof, 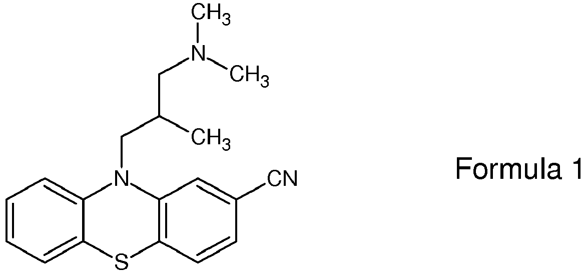 comprising:
   a) reacting 2-cyanophenthiazine of the formula-2 or salt thereof, with 1-dimethylamino-2-methyl-3-chloropropane of formula-3 or salt thereof, in the presence of base and phase transfer catalyst in organic solvent or mixture thereof, to produce the cyamemazine of formula-la 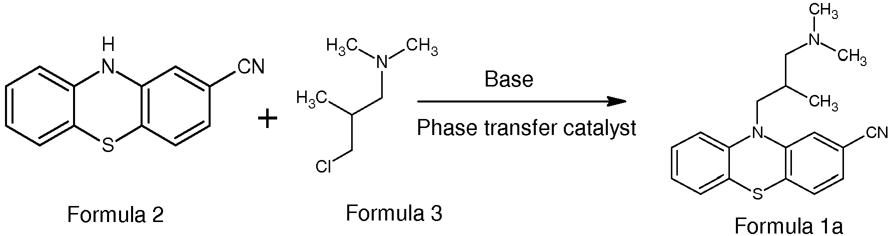
   b) reacting the compound of formula-la with an acid in organic solvent or mixture thereof, to obtain a cyamemazine acid salts
   c) optionally reacting with base to get cyamemazine
   d) optionally isolating or purifying the step (b) of cyamemazine acid salts or step (c) of cyamemazine is reacting with suitable salts to converting the cyamemazine of formula-1 or pharmaceutically acceptable salt thereof.
2. A process according to item 1, wherein the base is selected from the group comprising of organic or inorganic bases or a mixture thereof.
3. A process according to item 2, wherein the organic base is selected from the group comprising of amines such as triethylamine, diethylamine, benzylamine, pyridine, aniline or a mixture thereof.
4. A process according to item 2, wherein the inorganic base is selected from the group comprising of alkali metal hydroxides such as sodium, potassium or ammonium hydroxides and alkali metal carbonates such as sodium, potassium or ammonium carbonates alkali metal bi-carbonates such as sodium, potassium or ammonium bicarbonates or a mixture thereof.
5. A process according to item 2, wherein the base used is equimolar amount to about 1.5 to 2 times with respect to compound of formula-2.
6. A process according to item 1, wherein the phase transfer catalyst is selected from the group comprising of alkylammonium halides like tetrabutylammonium bromide, tetrapropylammonium bromide, benzyltributylammonium chloride, tetraethylammonium bromide, tetraoctylammonium bromide, benzyltrimethylammonium chloride, benzyl triethylammonium chloride, tetrabutylammonium iodide, tetrabutylammonium tribromide, tetrabutyl ammonium bromide, tetraheptylammonium bromide; alkylphosphonium halides like ethyltriphenylphosphonium bromide, ethyltriphenylphosphonium iodide, carboxy butyltriphenylphosphonium bromide, benzyltriphenylphosphonium bromide, benzyl triphenylphosphonium chloride; tetrabutylammonium hexafluorophosphate; tetra propylammonium hydroxide; tetrabutylammonium hydrogen sulfate; tetrabutyl ammonium acetate; cetylpyridinium chloride or a mixture thereof.
7. A process according to item 1, where in the condensation reaction is carried out for a period of time gaining from about 2 to 3 hours.
8. A process according to item 1, where in the condensation reaction is carried out at the temperature 100 to 110° C.
9. A process according to item 1, the compound of cyamemazine tartrate salt of formula-1c 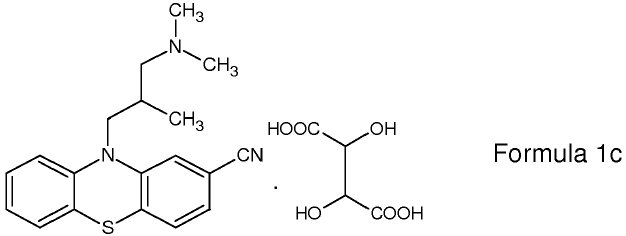 comprising:
   a) reacting the compound of cyamemazine maleate of formula-lb 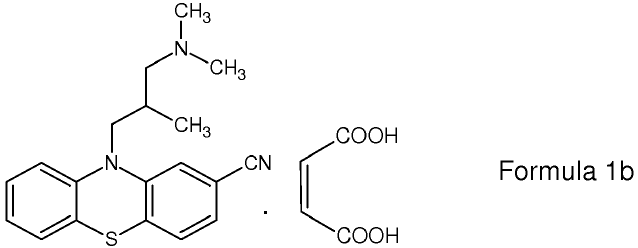 with a base in organic solvent or mixture thereof, to obtained a cyamemazine
   b) optionally isolating the cyamemazine and reacting with tartaric acid in organic solvent or mixture thereof, to obtain the cyamemazine tartrate salt and optionally purification with suitable organic solvents to get the pure cyamemazine tartrate salt of formula-1c.
10. A process according to item 1, the compound of cyamemazine tartrate salt of formula-1c 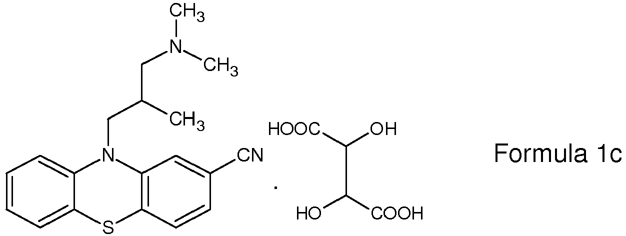 comprising:
   a) reacting the compound of cyamemazine of formula-1 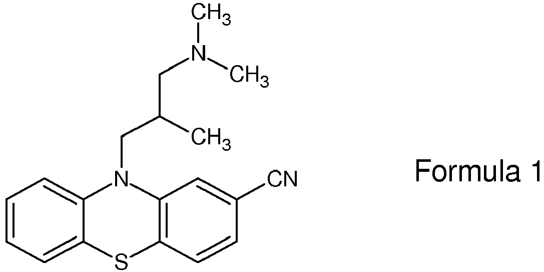 with tartaric acid in organic solvent or mixture thereof; to obtained the pure cyamemazine tartrate of formula-1c.
11. A process according to item 1, wherein the organic solvent is selected from the group comprising of alcohols, aliphatic hydrocarbons, aromatic hydrocarbons, halogenated hydrocarbons, ketones, ethers, esters, acetonitrile, dimethylformamide, dimethylacetamide dimethylsulfoxide, or a mixture thereof.
12. Cyamemazine or salts compound of formula-1 obtained according to item 1 is having the amount of each of the impurities X and Y in the level of less than 0.15% by HPLC.
13. Cyamemazine of formula-1 or salts compound obtained according to item 1 is having the purity greater than 99.50%; preferably 99.75%; more preferably 99.95% by HPLC.
14. A process according to item 1, the compound of cyamemazine of formula-1 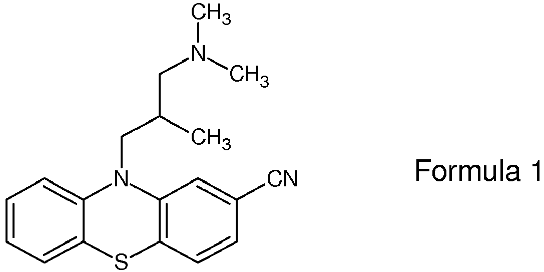 comprising:
   a) reacting the compound of cyamemazine tartrate salt of formula-1c 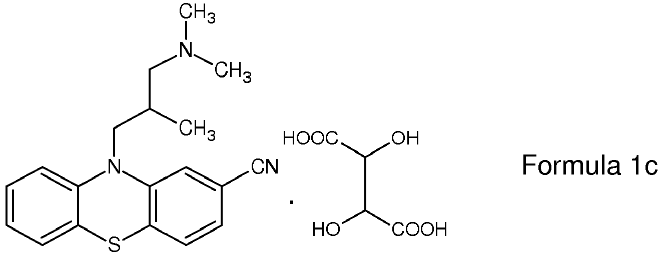 with a base in organic solvent or mixture thereof; to obtain pure cyamemazine of formula-1.
15. A process according to item 14, where in the solvent is acetone, di-isopropyl ether, methanol, toluene, ethyl acetate or mixture thereof.
16. Crystalline cyamemazine form C.
17. The crystalline form-C of item-16, characterized by a DSC thermogram shown in Fig-2 in combination with data selected from: a PXRD having peaks at 8.1, 10.4, 12.5, 16.4, 17.2, 18.3, 19.5, 24.8, 25.3, and 25.9 ± 0.2° 2-theta, a powder XRD substantially as depicted in Fig-1, and combinations thereof.
18. Crystalline cyamemazine tartrate form T.
19. The crystalline form-T of item-18, characterized by a DSC thermogram shown in Fig-6 in combination with data selected from: a PXRD having peaks at 10.6, 14.7, 16.8, 17.9, 20.4, 21.2, 22.8, 23.8 and 24.2 ± 0.2° 2-theta, a powder XRD substantially as depicted in Fig-5, and combinations thereof.
20. Compound of formula-X. 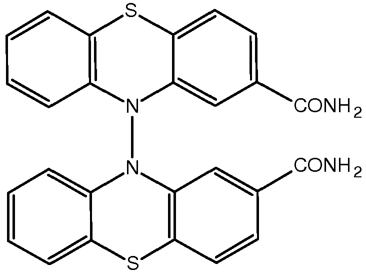
21. Compound of formula-Y. 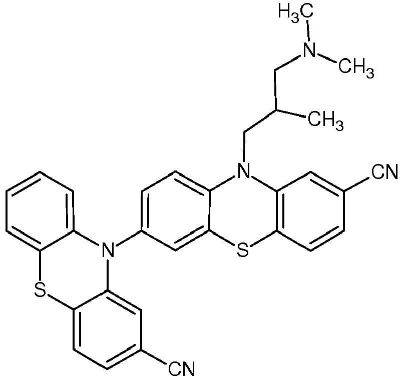
22. A process for the preparation of cyamemazine of formula-1, 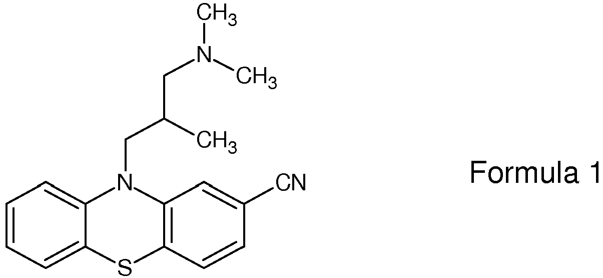 comprising:
   a) reacting 2-cyanophenthiazine of the formula-2 or salt thereof, with 1-dimethylamino-2-methyl-3-chloropropane of formula-3 or salt thereof, in the presence of base and phase transfer catalyst in organic solvent or mixture thereof, to produce the cyamemazine of formula-1. 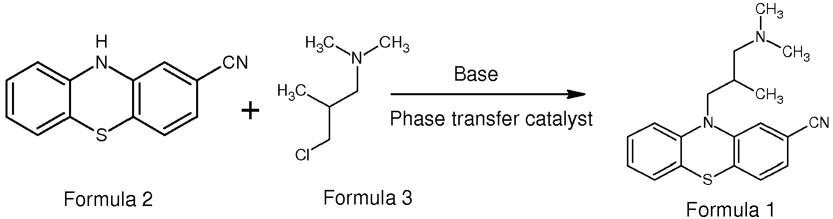
   b) optionally purifying the step (a) to get the cyamemazine of formula-1.

## Claims

1. A process for the preparation of Cyamemazine of formula-1 or pharmaceutically acceptable salts thereof, 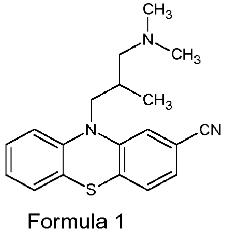 comprising:
(a) reacting 2-cyanophenthiazine of the formula-2 or salt thereof, with 1-dimethylamino-2-methyl-3-chloropropane of formula-3 or salt thereof, in the presence of a base and a phase transfer catalyst in an organic solvent, to produce crude cyamemazine of formula-1a 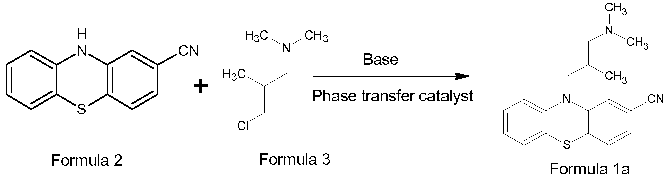
(b) reacting the compound of formula-la with an acid in an organic solvent, to obtain a Cyamemazine acid addition salt,
(c) converting the Cyamemazine acid addition salt obtained in step (b) to another Cyamemazine acid addition salt, or reacting the acid addition salts obtained in step (b) with a base to get pure Cyamemazine of formula-1, and
(d) optionally converting the Cyamemazine of formula-1 obtained in step (c) to Cyamemazine acid addition salts.

2. The process according to claim 1, wherein the base is selected from the group comprising of organic or inorganic bases or a mixture thereof, in equimolar amount to about 1.5 to 2 times with respect to compound of formula-2.

3. The process according to claim 2, wherein the organic base is selected from the group comprising of amines such as triethylamine, diethylamine, benzylamine, pyridine, aniline or a mixture thereof; and the inorganic base is selected from the group comprising of alkali metal hydroxides such as sodium, potassium or ammonium hydroxides and alkali metal carbonates such as sodium, potassium or ammonium carbonates alkali metal bi-carbonates such as sodium, potassium or ammonium bi-carbonates or a mixture thereof.

4. The process according to claim 1, wherein the phase transfer catalyst is selected from the group comprising of alkylammonium halides like tetrabutylammonium bromide, tetrapropylammonium bromide, benzyltributylammonium chloride, tetraethylammonium bromide, tetraoctylammonium bromide, benzyltrimethylammonium chloride, benzyl triethylammonium chloride, tetrabutylammonium iodide, tetrabutylammonium tribromide, tetrabutyl ammonium bromide, tetraheptylammonium bromide; alkylphosphonium halides like ethyltriphenylphosphonium bromide, ethyltriphenylphosphonium iodide, carboxy butyltriphenylphosphonium bromide, benzyltriphenylphosphonium bromide, benzyl triphenylphosphonium chloride; tetrabutylammonium hexafluorophosphate; tetra propylammonium hydroxide; tetrabutylammonium hydrogen sulfate; tetrabutyl ammonium acetate; cetylpyridinium chloride or a mixture thereof.

5. The process according to claim 1, where in the condensation reaction is carried out for a period of about 2 to 3 hours and at a temperature of 100 to 110° C.

6. The process according to claim 1, wherein the Cyamemezine acid addition salt obtained in step (c) is Cyamemazine tartrate salt of formula-lc, and is prepared by the process comprising: 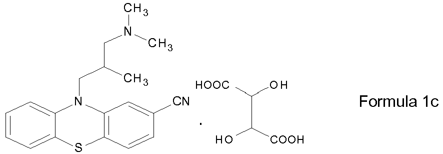
(a) reacting Cyamemazine maleate of formula-lb 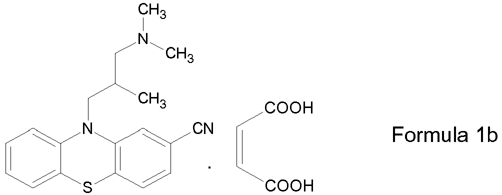 with a base in an organic solvent, to obtain a Cyamemazine of formula-1,
(b) optionally isolating the Cyamemazine of formula-1, and
(c) reacting the Cyamemazine of formula-1 with tartaric acid in an organic solvent, to obtain the Cyamemazine tartrate salt and optionally purifying with suitable organic solvents to get a pure Cyamemazine tartrate salt of formula-1c.

7. The process according to claim 1, wherein the Cyamemazine acid addition salt obtained in step (c) is Cyamemazine tartrate salt of formula-lc, the process comprising: 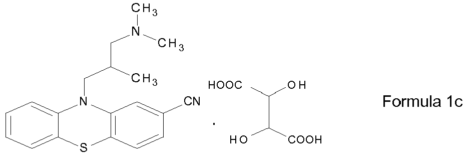
(a) reacting Cyamemazine maleate of formula-lb 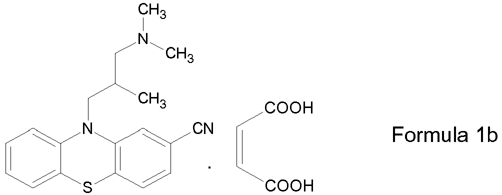 with tartaric acid in an organic solvent, to obtain a pure Cyamemazine tartrate of formula-1c.

8. The process according to any of the preceding claims, wherein the organic solvent is selected from the group comprising of alcohols, aliphatic hydrocarbons, aromatic hydrocarbons, halogenated hydrocarbons, ketones, ethers, esters, acetonitrile, dimethylformamide, dimethylacetamide, dimethylsulfoxide, or a mixture thereof.

9. The process according to any of the preceding claims, wherein the Cyamemazine and/or its salts has purity greater than 99.50% and less than 0.15% of impurities X and Y, by HPLC.

10. The process according to claim 1, wherein the pure Cyamemazine of formula-1 in step (c) is obtained by the process comprising: 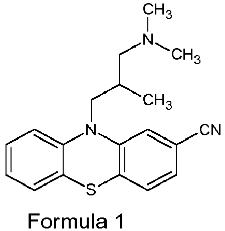
(a) reacting Cyamemazine tartrate salt of formula-1c 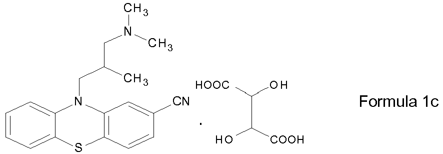 with a base in an organic solvent or mixture thereof, to obtain pure Cyamemazine of formula-1.

11. The process according to claim 10, wherein the organic solvent is acetone, di-isopropyl ether, methanol, toluene, ethyl acetate or a mixture thereof.

12. The process according to claim 1, wherein Cyamemazine is in Crystalline form C, **characterized by** a DSC thermogram having an onset temperature of about 90°C in combination with data selected from: a PXRD having peaks at 8.1, 10.4, 12.5, 16.4, 17.2, 18.3, 19.5, 24.8, 25.3, and 25.9 ± 0.2° 2-theta, a powder XRD substantially as depicted in Fig-1, and combinations thereof.

13. The process according to claim 1, wherein the Cyamemazine acid addition salt obtained in step (c) is Cyamemazine tartrate in crystalline form T, **characterized by** a DSC thermogram having an onset temperature of about 182°C in combination with data selected from: a PXRD having peaks at 10.6, 14.7, 16.8, 17.9, 20.4, 21.2, 22.8, 23.8 and 24.2 ± 0.2° 2-theta, a powder XRD substantially as depicted in Fig-5, and combinations thereof.

14. Compound of formula-X

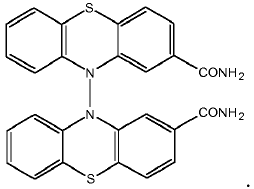

15. Compound of formula-Y

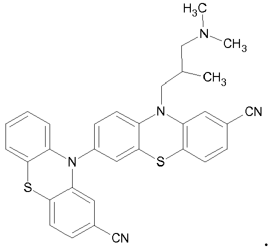

## Patentansprüche

1. Verfahren zur Herstellung von Cyamemazin der Formel 1 oder pharmazeutisch annehmbaren Salzen davon, 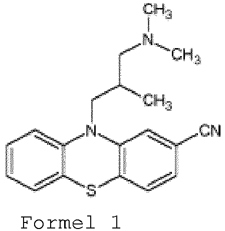 umfassend:
(a) Reagieren von 2-Cyanophenthiazin der Formel 2 oder eines Salzes davon mit 1-Dimethylamino-2-methyl-3-chlorpropan der Formel 3 oder eines Salzes davon, in der Gegenwart einer Base und eines Phasentransferkatalysators in einem organischen Lösungsmittel, um Rohcyamemazin der Formel 1a zu erzeugen 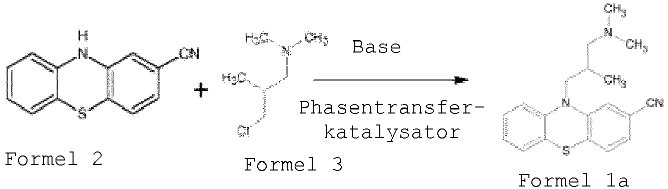
(b) Reagieren der Verbindung der Formel 1a mit einer Säure in einem organischen Lösungsmittel, um ein Cyamemazinsäureadditionssalz zu erhalten,
(c) Umwandeln des in Schritt (b) erhaltenen Cyamemazinsäureadditionssalzes in ein anderes Cyamemazinsäureadditionssalz oder Reagieren der in Schritt (b) erhaltenen Säureadditionssalze mit einer Base, um reines Cyamemazin der Formel 1 zu erhalten, und
(d) optionales Umwandeln des in Schritt (c) erhaltenen Cyamemazins der Formel 1 in Cyamemazinsäureadditionssalze.

2. Verfahren nach Anspruch 1, wobei die Base aus der Gruppe ausgewählt ist, die organische oder anorganische Basen oder ein Gemisch davon umfasst, in einer äquimolaren Menge bis ungefähr dem 1,5- bis 2-Fachen in Bezug auf die Verbindung der Formel 2.

3. Verfahren nach Anspruch 2, wobei die organische Base aus der Gruppe ausgewählt ist, die Amine wie zum Beispiel Triethylamin, Diethylamin, Benzylamin, Pyridin, Anilin oder ein Gemisch davon umfasst; und die anorganische Base aus der Gruppe ausgewählt ist, die Alkalimetallhydroxide wie zum Beispiel Natrium-, Kalium- oder Ammoniumhydroxide und Alkalimetallkarbonate wie zum Beispiel Natrium-, Kalium- oder Ammoniumkarbonate, Alkalimetallbikarbonate wie zum Beispiel Natrium-, Kalium- oder Ammoniumbikarbonate oder ein Gemisch davon umfasst.

4. Verfahren nach Anspruch 1, wobei der Phasentransferkatalysator aus der Gruppe ausgewählt ist, die aus Alkylammoniumhaliden wie Tetrabutylammoniumbromid, Tetrapropylammoniumbromid, Benzyltributylammoniumchlorid, Tetraethylammoniumbromid, Tetraoctylammoniumbromid, Benzyltrimethylammoniumchlorid, Benzyltriethylammoniumchlorid, Tetrabutylammoniumiodid, Tetrabutylammoniumtribromid, Tetrabutylammoniumbromid, Tetraheptylammoniumbromid; Alkylphosphoniumhaliden wie Ethyltriphenylphosphoniumbromid, Ethyltriphenylphosphoniumiodid, Carboxybutyltriphenylphosphoniumbromid, Benzyltriphenylphosphoniumbromid, Benzyltriphenylphosphoniumchlorid; Tetrabutylammoniumhexafluorphosphat; Tetrapropylammoniumhydroxid; Tetrabutylammoniumhydrogensulfat; Tetrabutylammoniumacetat; Cetylpyridiniumchlorid oder einem Gemisch davon besteht.

5. Verfahren nach Anspruch 1, wobei die Kondensationsreaktion über einen Zeitraum von ungefähr 2 bis 3 Stunden und bei einer Temperatur von 100 bis 110 °C durchgeführt wird.

6. Verfahren nach Anspruch 1, wobei das in Schritt (c) erhaltene Cyamemazinsäureadditionssalz Cyamemazintartratsalz der Formel 1c ist und durch das Verfahren hergestellt wird, das Folgendes umfasst: 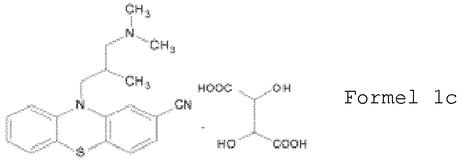
(a) Reagieren von Cyamemazinmaleat der Formel 1b 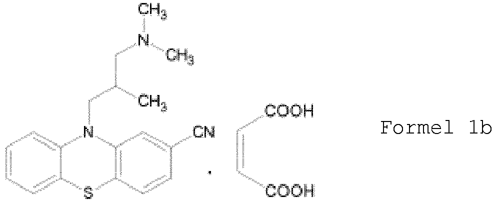 mit einer Base in einem organischen Lösungsmittel, um ein Cyamemazin der Formel 1 zu erhalten,
(b) optionales Isolieren des Cyamemazins der Formel 1, und
(c) Reagieren des Cyamemazins der Formel 1 mit Weinsäure in einem organischen Lösungsmittel, um das Cyamemazintartratsalz zu erhalten, und optionales Reinigen mit geeigneten organischen Lösungsmitteln, um ein reines Cyamemazintartratsalz der Formel 1c zu erhalten.

7. Verfahren nach Anspruch 1, wobei das in Schritt (c) erhaltene Cyamemazinsäureadditionssalz Cyamemazintartratsalz der Formel 1c ist, wobei das Verfahren Folgendes umfasst: 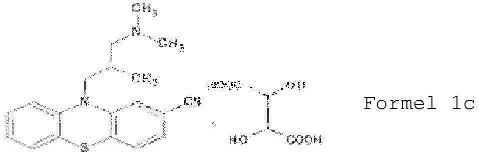
(a) Reagieren von Cyamemazinmaleat der Formel 1b 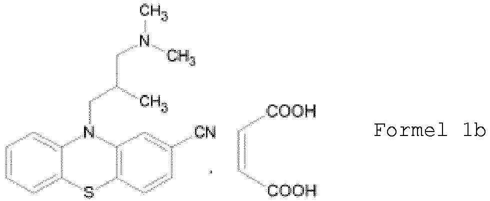 mit Weinsäure in einem organischen Lösungsmittel, um ein reines Cyamemazintartrat der Formel 1c zu erhalten.

8. Verfahren nach einem der vorhergehenden Ansprüche, wobei das organische Lösungsmittel aus der Gruppe ausgewählt ist, die Alkohole, aliphatische Kohlenwasserstoffe, aromatische Kohlenwasserstoffe, halogenierte Kohlenwasserstoffe, Ketone, Ether, Ester, Acetonitril, Dimethylformamid, Dimethylacetamid, Dimethylsulfoxid oder ein Gemisch davon umfasst.

9. Verfahren nach einem der vorhergehenden Ansprüche, wobei das Cyamemazin und/oder seine Salze eine Reinheit von mehr als 99,50 % und weniger als 0,15 % an Unreinheiten X und Y durch HPLC aufweisen.

10. Verfahren nach Anspruch 1, wobei das reine Cyamemazin der Formel 1 in Schritt (c) durch das Verfahren erhalten wird, das Folgendes umfasst: 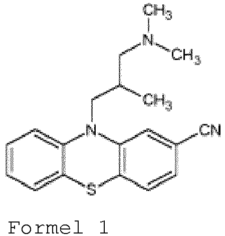
(a) Reagieren von Cyamemazintartratsalz der Formel 1c 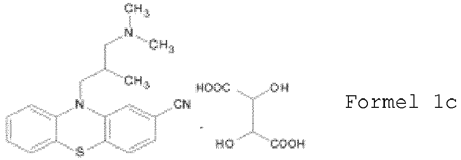 mit einer Base in einem organischen Lösungsmittel oder Gemisch davon, um reines Cyamemazin der Formel 1 zu erhalten.

11. Verfahren nach Anspruch 10, wobei das organische Lösungsmittel Aceton, Diisopropylether, Methanol, Toluol, Ethylacetat oder ein Gemisch davon ist.

12. Verfahren nach Anspruch 1, wobei Cyamemazin in kristalliner Form C ist, **gekennzeichnet durch** ein DSC-Thermogramm, das eine Anfangstemperatur von ungefähr 90 °C in Kombination mit Daten ausgewählt aus dem Folgenden aufweist: einer PXRD, die Spitzen bei 8,1, 10,4, 12,5, 16,4, 17,2, 18,3, 19,5, 24,8, 25,3 und 25,9 ± 0,2° 2-theta aufweist, einer Pulver-XRD, die im Wesentlichen wie in Fig. 1 dargestellt ist und Kombinationen davon.

13. Verfahren nach Anspruch 1, wobei das in Schritt (c) erhaltene Cyamemazinsäureadditionssalz Cyamemazintartrat in kristalliner Form T ist, **gekennzeichnet durch** ein DSC-Thermogramm, das eine Anfangstemperatur von ungefähr 182 °C in Kombination mit Daten ausgewählt aus dem Folgenden aufweist: einer PXRD, die Spitzen bei 10,6, 14,7, 16,8, 17,9, 20,4, 21,2, 22,8, 23,8 und 24,2 ± 0,2° 2-theta aufweist, einer Pulver-XRD, die im Wesentlichen wie in Fig. 5 dargestellt ist und Kombinationen davon.

14. Verbindung der Formel X

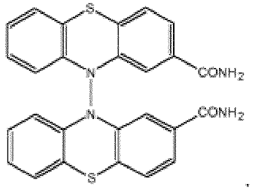

15. Verbindung der Formel Y

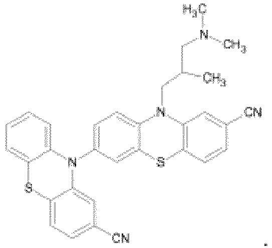

## Revendications

1. Procédé pour la préparation de cyamémazine de formule 1 ou de ses sels pharmaceutiquement acceptables, 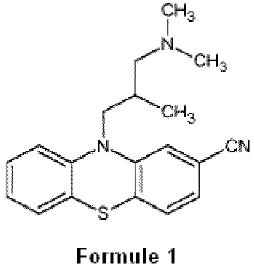 comprenant :
(a) la réaction de la 2-cyanophenthiazine de la formule 2 ou de l'un de ses sels, avec du 1-diméthylamino-2-méthyl-3-chloropropane de formule 3 ou l'un de ses sels, en présence d'une base et d'un catalyseur de transfert de phase dans un solvant organique, pour produire de la cyamémazine brute de formule la 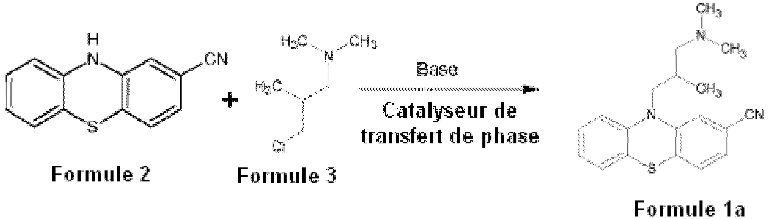
(b) la réaction du composé de formule la avec un acide dans un solvant organique, pour obtenir un sel d'addition d'acide de cyamémazine,
(c) la conversion du sel d'addition d'acide de cyamémazine obtenu dans l'étape (b) en un autre sel d'addition d'acide de cyamémazine, ou la réaction des sels d'addition d'acide obtenus dans l'étape (b) avec une base pour obtenir la cyamémazine pure de formule 1, et
(d) la conversion éventuelle de la cyamémazine de formule 1 obtenue dans l'étape (c) en sels d'addition d'acide de cyamémazine.

2. Procédé selon la revendication 1, dans lequel la base est choisie dans le groupe composé de bases organiques ou inorganiques ou de l'un de leurs mélanges, dans une quantité équimolaire jusqu'à environ 1,5 à 2 fois par rapport au composé de formule 2.

3. Procédé selon la revendication 2, dans lequel la base organique est choisie dans le groupe composé d'amines telles que la triéthylamine, la diéthylamine, la benzylamine, la pyridine, l'aniline ou l'un de leurs mélanges ; et la base inorganique est choisie dans le groupe composé d'hydroxydes de métaux alcalins tels que des hydroxydes de sodium, de potassium ou d'ammonium et des carbonates de métaux alcalins tels que des carbonates de sodium, de potassium ou d'ammonium, des bicarbonates de métaux alcalins tels que des bicarbonates de sodium, de potassium ou d'ammonium ou l'un de leurs mélanges.

4. Procédé selon la revendication 1, dans lequel le catalyseur de transfert de phase est choisi dans le groupe composé d'halogénures d'alkylammonium tels que le bromure de tétrabutylammonium, le bromure de tétrapropyl-ammonium, le chlorure de benzyltributylammonium, le bromure de tétraéthylammonium, le bromure de tétra-octylammonium, le chlorure de benzyltriméthylammonium, le chlorure de benzyltriéthylammonium, l'iodure de tétrabutylammonium, le tribromure de tétrabutylammonium, le bromure de tétrabutylammonium, le bromure de tétraheptylammonium ; d'halogénures d'alkylphosphonium comme le bromure d'éthyltriphénylphosphonium, l'iodure d'éthyltriphénylphosphonium, le bromure de carboxybutyltriphénylphosphonium, le bromure de benzyltriphénylphosphonium, le chlorure de benzyltriphénylphosphonium ; l'hexafluorophosphate de tétrabutylammonium ; l'hydroxyde de tétrapropylammonium ; l'hydrogénosulfate de tétrabutylammonium ; l'acétate de tétrabutylammonium ; le chlorure de cétylpyridinium ou l'un de leurs mélanges.

5. Procédé selon la revendication 1, dans lequel la réaction de condensation est réalisée pendant une période d'environ 2 à 3 heures et à une température de 100 à 110 °C.

6. Procédé selon la revendication 1, dans lequel le sel d'addition d'acide de cyamémazine obtenu dans l'étape (c) est le sel tartrate de cyamémazine de formule 1c, et est préparé par le procédé comprenant : 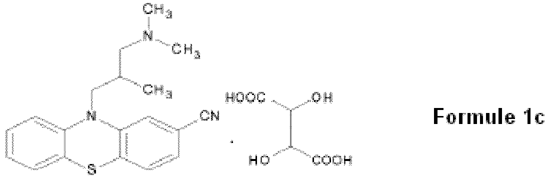
(a) la réaction du maléate de cyamémazine de formule 1b 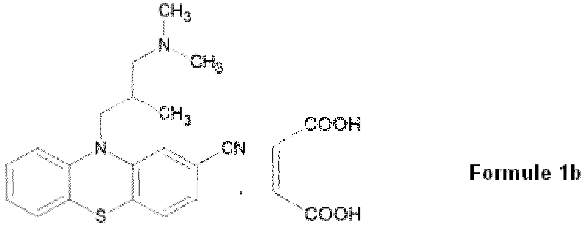 avec une base dans un solvant organique, pour obtenir une cyamémazine de formule 1,
(b) l'isolation éventuelle de la cyamémazine de formule 1, et
(c) la réaction de la cyamémazine de formule 1 avec de l'acide tartrique dans un solvant organique, pour obtenir le sel tartrate de cyamémazine et éventuellement la purification avec des solvants organiques appropriés pour obtenir un sel tartrate de cyamémazine pur de formule 1c.

7. Procédé selon la revendication 1, dans lequel le sel d'addition d'acide de cyamémazine obtenu dans l'étape (c) est le sel tartrate de cyamémazine de formule 1c, le procédé comprenant : 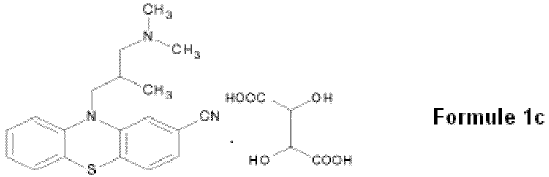
(a) la réaction du maléate de cyamémazine de formule 1b 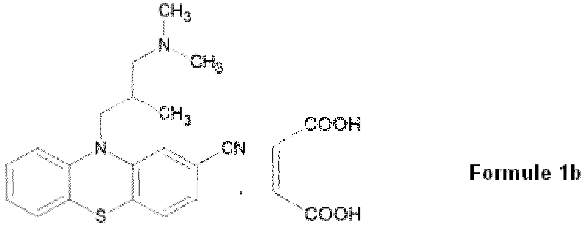 avec de l'acide tartrique dans un solvant organique, pour obtenir un tartrate de cyamémazine pur de formule 1c.

8. Procédé selon l'une quelconque des revendications précédentes, dans lequel le solvant organique est choisi dans le groupe composé d'alcools, d'hydrocarbures aliphatiques, d'hydrocarbures aromatiques, d'hydrocarbures halogénés, de cétones, d'éthers, d'esters, d'acétonitrile, de diméthylformamide, de diméthyl-acétamide, de sulfoxyde de diméthyle, ou de l'un de leurs mélanges.

9. Procédé selon l'une quelconque des revendications précédentes, dans lequel la cyamémazine et/ou ses sels présentent une pureté supérieure à 99,50 % et moins de 0,15 % d'impuretés X et Y, par CLHP.

10. Procédé selon la revendication 1, dans lequel la cyamémazine pure de formule 1 dans l'étape (c) est obtenue par le procédé comprenant : 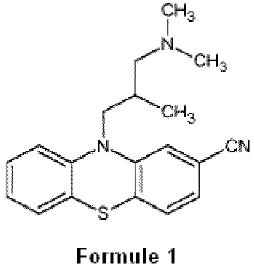
(a) la réaction du sel tartrate de cyamémazine de formule 1c 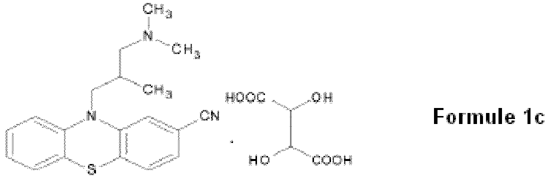 avec une base dans un solvant organique ou l'un de ses mélanges, pour obtenir la cyamémazine pure de formule 1.

11. Procédé selon la revendication 10, dans lequel le solvant organique est l'acétone, l'éther diisopropylique, le méthanol, le toluène, l'acétate d'éthyle ou l'un de leurs mélanges.

12. Procédé selon la revendication 1, dans lequel la cyamémazine est sous forme cristalline C, **caractérisée par** un thermogramme de DSC présentant une température d'apparition d'environ 90 °C en combinaison avec des données choisies parmi : un PXRD présentant des pics à 8,1, 10,4, 12,5, 16,4, 17,2, 18,3, 19,5, 24,8, 25,3, et 25,9 ± 0,2° 2-thêta, un XRD sur poudre sensiblement tel qu'illustré sur la figure 1, et leurs combinaisons.

13. Procédé selon la revendication 1, dans laquelle le sel d'addition d'acide de cyamémazine obtenu dans l'étape (c) est le sel tartrate de cyamémazine sous forme cristalline T, **caractérisée par** un thermogramme de DSC présentant une température d'apparition d'environ 182 °C en combinaison avec des données choisies parmi : un PXRD présentant des pics à 10,6, 14,7, 16,8, 17,9, 20,4, 21,2, 22,8, 23,8, et 24,2 ± 0,2° 2-thêta, un XRD sur poudre sensiblement tel qu'illustré sur la figure 5, et leurs combinaisons.

14. Composé de formule X

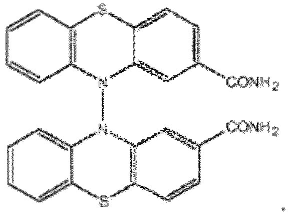

15. Composé de formule Y
